(19) Europäisches Patentamt
European Patent Office
Office européen des brevets

(11) Veröffentlichungsnummer: **0 250 833**
**A1**

(12) # EUROPÄISCHE PATENTANMELDUNG

(21) Anmeldenummer: 87107379.7

(51) Int. Cl.⁴: **A61F 13/14**

(22) Anmeldetag: 21.05.87

(30) Priorität: 25.06.86 DE 3621287

(43) Veröffentlichungstag der Anmeldung:
07.01.88 Patentblatt 88/01

(84) Benannte Vertragsstaaten:
AT BE CH DE ES FR GB GR IT LI NL SE

(71) Anmelder: S + G IMPLANTS GMBH
Grapengiesserstrasse 34
D-2400 Lübeck(DE)

(72) Erfinder: Henssge, Joachim, Prof. Dr.
Im Trentsaal 7
D-2400 Lübeck(DE)
Erfinder: Grundei, Hans
Gärtnergasse 4
D-2400 Lübeck(DE)

(74) Vertreter: Wilcken, Thomas, Dipl.-Ing. et al
Musterbahn 1
D-2400 Lübeck(DE)

(54) Stützendes Mieder.

(57) Ein stützendes Mieder aus elastischem Mieder (1), welches im angelegten Zustand den Körper (2) eines Menschen zumindest vom oberen Hüftbereich bis zum Brustansatz umschließt, wird zur Verstärkung der stützenden Wirkung mit einer vorteilhaft elastischen Binde (3) kombiniert, deren Länge so groß gewählt ist, daß sie von unten ausgehend wendelförmig um das an den Körper (2) angelegte Mieder (1) wickelbar ist.

## Stützendes Mieder

Die Erfindung bezieht sich auf ein stützendes Mieder aus elastischem Material, welches im angelegten Zustand den Körper eines Menschen zumindest vom oberen Hüftbereich bis zum Brustansatz umschließt.

In einigen Fällen und insbesondere dann, wenn von einem Träger des Mieders schwere Lasten zu heben oder zu tragen sind oder für Patienten mit sehr schwacher Körperstabilität reicht die Stützwirkung eines bekannten, elastischen Mieders häufig nicht aus.

Die Aufgabe der Erfindung besteht darin, die Stützwirkung elastischer Mieder auf einfache Weise erheblich zu verstärken.

Die Lösung dieser Aufgabe kennzeichnet sich bei dem stützenden Mieder der eingangs erwähnten Art durch eine Binde, deren Länge so groß gewählt ist, daß sie von unten ausgehend wendelförmig um das an den Körper angelegte Mieder wickelbar ist.

Durch diese Lösung ist ein Patient in der Lage, ohne Schwie rigkeiten den Körper aufrechtzuerhalten, und ein Mensch, der schwerste Lasten zu tragen hat, ist durch diese Lösung gegen Schäden im Rücken, insbesondere an der Wirbelsäule, geschützt.

Man geht nach der Erfindung als weitere Ausbildung so vor, daß das Ende der Binde lösbar unten, z.B. durch einen Klettenverschluß, am Mieder zu befestigen ist und daß die Binde vorteilhaft aus einem elastischen Material besteht.

Es hat sich gezeigt, daß die Binde bei einer Breite von 5 bis 15 cm eine Länge von 300 bis 500 cm aufweist, und zwar je nach Größe des Trägers.

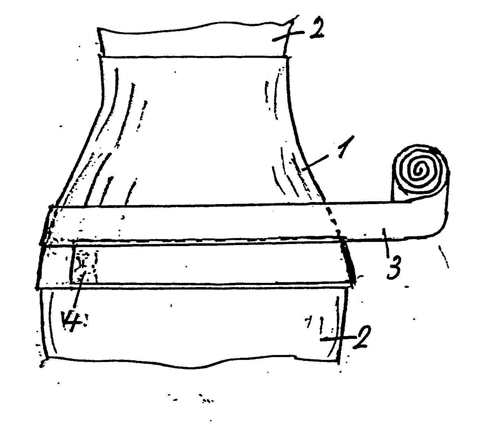

Das an einen menschlichen Körper anzulegende Mieder ist in der Zeichnung und die mit zwei Wicklungen um das Mieder gewickelte Binde schematisch in Ansicht dargestellt.

Es ist bekannt, an den Körper 2 von Patienten oder von Menschen, die Lasten heben oder tragen müssen, ein elastisches Stützmieder 1 anzulegen, welches etwa vom oberen Hüftbereich bis zum Brustansatz verläuft und dadurch die Stabilität des Körpers, und insbesondere der Wirbelsäule verstärkt.

Nach der Erfindung wird der Körper durch eine vorteilhaft elastische Binde 3 in Kombination mit dem elastischen Mieder zusätzlich unterstützt, deren Länge so groß gewählt ist, daß sie von unten ausgehend wendelförmig um das an den Körper angelegte Mieder wickelbar ist.

Vorteilhaft beträgt die Bindenlänge entsprechend der Größe des Trägers 300 bis 500 cm bei einer Breite zwischen 5-15 cm. Dabei kann die Binde mit dem einen Ende unten mit dem Mieder 1 bis 4 lösbar verbunden sein, und das Ende der Binde wird nach dem Umwickeln um das Mieder in einer bekannten Weise, z.B. durch einen Klettenverschluß, befestigt. Es ist auch möglich, das Mieder selbst auf der Rückseite mit Taschen zu versehen, die Versteifungen, z.B. Versteifungsstäbe, aufnehmen, um dadurch eine weitere Stützung des Körpers zu erreichen.

## Ansprüche

1. Stützendes Mieder aus elastischem Material, welches im angelegten Zustand den Körper eines Menschen zumindest vom oberen Hüftbereich bis zum Brustansatz umschließt, gekennzeichnet durch eine Binde (2), deren Länge so groß gewählt ist, daß sie von unten ausgehend wendelförmig um das an den Körper angelegte Mieder (1) wickelbar ist.

2. Mieder nach Anspruch 1, dadurch gekennzeichnet, daß das eine Ende der Binde lösbar unten am Mieder befestigt ist.

3. Mieder nach einem der Ansprüche 1 und 2, dadurch gekennzeichnet, daß die Binde aus elastischem Material besteht.

4. Mieder nach einem der Ansprüche 1 bis 3, dadurch gekennzeichnet, daß die Binde eine Länge von 300 bis 500 cm und eine Breite von 5 bis 15 cm hat.

5. Mieder nach einem der Ansprüche 1 bis 4, dadurch gekennzeichnet, daß an seiner Rückseite Taschen zur Aufnahme von festen Stützteilen vorgesehen sind.

| | Europäisches Patentamt | **EUROPÄISCHER RECHERCHENBERICHT** | Nummer der Anmeldung |
|---|---|---|---|
| | | | EP 87 10 7379 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | US-A-3 920 008 (I.S. LEHMAN)<br>* Spalte 2, Zeile 37 - Spalte 4, Zeile 16; Abbildungen 1-5 *<br><br>--- | 1-5 | A 61 F 13/14 |
| Y | US-A-2 058 040 (S.R. SEVERN)<br>* Seite 1, Spalte 2, Zeilen 37-49; Abbildungen *<br><br>--- | 1-5 | |
| A | US-A-3 529 601 (J.W. KIRKLAND)<br>* Spalte 2, Zeile 47 - Spalte 3, Zeile 26; Spalte 3, Zeilen 48-71; Abbildungen *<br><br>--- | 1,3,4 | |
| A | GB-A- 104 255 (M. BERRE)<br>* Abbildungen 6-9 *<br><br>--- | 1,4 | |
| A | DE-C- 445 382 (P.C.A. MESTREAU et al.)<br>* Seite 2, Zeilen 86-100; Abbildungen 1-3 *<br><br>--- | 1,4 | RECHERCHIERTE SACHGEBIETE (Int. Cl.4)<br><br>A 61 F<br>A 41 C |
| A | FR-A- 713 107 (ETABLISSEMENTS M. MARIE)<br>* Insgesamt *<br><br>--- | 1,3 | |
| A | US-A-3 298 366 (E.S. MOORE et al.)<br>* Spalte 1, Zeilen 49-61; Abbildungen *<br><br>--- -/- | 1-5 | |

Der vorliegende Recherchenbericht wurde für alle Patentansprüche erstellt.

| Recherchenort | Abschlußdatum der Recherche | Prüfer |
|---|---|---|
| DEN HAAG | 30-09-1987 | WOLF C.H.S. |

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US-A-2 680 848 (L. HANNES et al.)<br>* Spalte 3, Zeilen 20-25; Zeile 58 - Spalte 4, Zeile 4; Abbildungen *<br><br>----- | 1-3 | |
| | | | **RECHERCHIERTE SACHGEBIETE (Int. Cl.4)** |

Der vorliegende Recherchenbericht wurde für alle Patentansprüche erstellt.

| Recherchenort | Abschlußdatum der Recherche | Prüfer |
|---|---|---|
| DEN HAAG | 30-09-1987 | WOLF C.H.S. |